# EUROPÄISCHE PATENTANMELDUNG

(11) **EP 4 806 430 A2**
(43) Veröffentlichungstag der Anmeldung: **16.09.2026**
(21) Anmeldenummer: 26180520.4
(22) Anmeldetag: 22.05.2026
(51) Int. Cl.: A61K 31/198, A61K 31/7004, A61K 31/28, A61K 31/353, A61K 31/375, A61K 31/59, A61K 31/525, A61K 31/4415, A61K 31/714, A61P 13/00, A61P 31/00

(54) **ZUSAMMENSETZUNG ZUR VORBEUGUNG VON HARNWEGSINFEKTIONEN**

(71) Anmelder: Dr. August Wolff GmbH & Co. KG, 33611 Bielefeld (DE)
(72) Erfinder: SCHULZE ZUR WIESCHE, Erik, 33611 Bielefeld (DE); HÄUSER, Manuel, 32105 Bad Salzuflen (DE); KELM, Maren, 33790 Halle (DE)
(74) Vertreter: Weickmann & Weickmann PartmbB

(57) **Zusammenfassung**

Die vorliegende Erfindung betrifft eine Zusammensetzung zur oralen Anwendung umfassend Methionin und Dimethylglycin und/oder ein Salz von Dimethylglycin, eine Lösung umfassend die erfindungsgemäße Zusammensetzung, sowie die Zusammensetzung oder Lösung zur Verwendung bei der Vorbeugung und/oder Behandlung von Infektionen und/oder Entzündungen der Harnwege.

## Beschreibung

Die vorliegende Erfindung betrifft eine Zusammensetzung zur oralen Anwendung umfassend Methionin und Dimethylglycin und/oder ein Salz von Dimethylglycin, eine Lösung umfassend die erfindungsgemäße Zusammensetzung, sowie die Zusammensetzung oder Lösung zur Verwendung bei der Vorbeugung und/oder Behandlung von Infektionen und/oder Entzündungen der Harnwege.

Harnwegsinfektionen (HWI) sind Infektionen der ableitenden Harnwege, welche überwiegend durch Bakterien verursacht werden. Typische Symptome einer Harnwegsinfektion sind Schmerzen oder Brennen beim Wasserlassen sowie ein häufiger und starker Harndrang. Bakterielle Harnwegsinfektionen zählen zu den häufigsten bakteriellen Infektionen des Menschen und stellen eine erhebliche medizinische und gesundheitsökonomische Belastung dar.

Es wird unterschieden zwischen oberen Harnwegsinfektionen, insbesondere Pyelonephritis (Nierenbeckenentzündungen), und unteren Harnwegsinfektionen, zu denen die Zystitis (Blasenentzündung) und Urethritis (Harnröhrenentzündung) zählen. Zu den häufigsten Erregern zählen Enterobakterien, insbesondere *Escherichia coli,* aber auch Klebsiellen oder *Proteus*-Arten. Häufig entstehen Infekte durch aszendierende Erreger, welche ausgehend von einer initialen äußeren Schmierinfektion an der Harnröhrenöffnung durch die Harnröhre aufsteigen und sich in der Harnblase vermehren.

Harnwegsinfektionen treten bei beiden Geschlechtern, jedoch deutlich häufiger bei Frauen auf, was sich anatomisch durch die kürzere weibliche Harnröhre und die Nähe zwischen äußerer Harnröhrenöffnung und Anus erklären lässt. Es wird vermutet, dass etwa 60% aller Frauen mindestens einmal im Leben eine symptomatische Harnwegsinfektion entwickeln (Turcu et al., J. Mind Med. Sci. 2025, 12(1),5). Ab zwei Infektionen pro Halbjahr oder drei Infektionen pro Jahr spricht man von einer rezidivierenden Harnwegsinfektion.

Obwohl die meisten Harnwegsinfektionen gut mitAntibiotika behandelt werden können, stellen insbesondere rezidivierende Harnwegsinfektionen eine erhebliche Belastung für die Patientinnen dar. Auch können sich durch die häufige Antibiotikagabe leicht Resistenzen ausbilden, welche die Behandlungsmöglichkeiten rezidivierender Harnwegsinfektionen zusätzlich einschränken und den Leidensdruck weiter erhöhen.

Es besteht daher dringend Bedarf an wirksamen prophylaktischen Verfahren und Mitteln zur Vorbeugung von Harnwegsinfektionen, aber auch an Mitteln, die bei der Behandlung leichter Harnwegsinfektionen oder ergänzend und unterstützend zu Antibiotikatherapien eingesetzt werden können. Um eine kontinuierliche vorbeugende Einnahme über einen längeren Zeitraum zu erlauben, sollten diese Mittel leicht anwendbar und möglichst frei von Nebenwirkungen sein.

Es hat sich gezeigt, dass diese Aufgabe durch die vorliegende erfindungsgemäße Zusammensetzung überraschend gelöst wird.

In einem ersten Aspekt betrifft die vorliegende Erfindung eine Zusammensetzung zur oralen Anwendung, umfassend:
(A) Methionin, und
(B) Dimethylglycin und/oder ein Salz von Dimethylglycin.

Die erfindungsgemäße Zusammensetzung ist zur oralen Anwendung vorgesehen. Wie hierin verwendet, ist unter oraler Anwendung insbesondere die perorale Einnahme der Zusammensetzung zu verstehen. Bevorzugt sind alle Inhaltsstoffe der Zusammensetzung für Menschen ungiftig und zur oralen Einnahme geeignet. In bevorzugten Ausführungsformen sind alle Wirkstoffe und/oder alle Inhaltsstoffe der erfindungsgemäßen Zusammensetzung pharmazeutisch annehmbare Substanzen.

Methionin ist eine schwefelhaltige α-Aminosäure und wird durch die folgende chemische Formel (I) dargestellt:

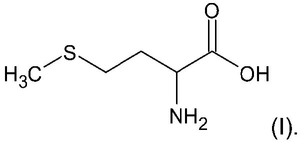

Das L-Enantiomer von Methionin, wie dargestellt in Formel (la), ist eine essentielle, proteinogene Aminosäure.

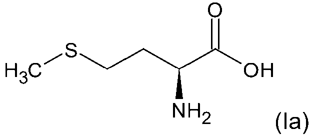

Erfindungsgemäß kann es sich bei dem Methionin (A) um L-Methionin, D-Methionin oder eine beliebige Mischung dieser Enantiomere handeln. Bevorzugt ist das Methionin (A) L-Methionin. Methionin kann in der Zusammensetzung als reines Methionin oder als Solvat, Hydrat oder Salz von Methionin, bevorzugt als reines Methionin oder als pharmazeutisch annehmbares Salz von Methionin, besonders bevorzugt als reines Methionin vorliegen.

Es ist bekannt, dass überschüssiges Methionin im Körper hauptsächlich über Homocystein zu Cystein metabolisiert wird. Durch Oxidation des Schwefels im Cystein oder in anderen schwefelhaltigen Metaboliten entstehen Sulfat und Protonen, welche über den Urin ausgeschieden werden. Somit führt die Einnahme von Methionin effektiv zu einer Ansäuerung des Harns. Ohne an eine Theorie gebunden zu sein, wird vermutet, dass durch die Ansäuerung des Urins das Wachstum pathogener Bakterien gehemmt und die Adhäsion pathogener Bakterien an den Harnwegsepithelien unterdrückt werden kann. Ein saures Milieu kann außerdem die Wirkung verschiedener Antibiotika verbessern, welche bei niedrigem pH-Wert besonders wirksam sind. Darüber hinaus kann Methionin über seine Metabolite zur Aufrechterhaltung des körpereigenen antioxidativen Systems beitragen, was ferner zu seiner positiven Wirkung beitragen kann.

Bevorzugt enthält die erfindungsgemäße Zusammensetzung 0,0001-25 Gew.-%, bevorzugt 0,01-20 Gew.-%, stärker bevorzugt 1-10 Gew.-% Methionin (A), insbesondere L-Methionin. Wie hierin verwendet, beziehen sich Gewichtsprozente (Gew.-%) stets auf das Gesamtgewicht der Zusammensetzung, wenn nicht anders angegeben.

Dimethylglycin (N,N-Dimethylglycin, DMG) kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein-Methylase.

N,N-Dimethylglycin, auch Dimethylaminoessigsäure (DMG), wird durch die folgende chemische Formel (II) dargestellt:

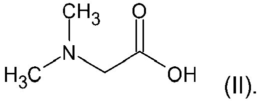

Erfindungsgemäß ist nicht nur der Einsatz von Dimethylglycin, sondern auch der von dessen Salzen, Solvaten und Hydraten. Dabei handelt es sich bevorzugt um pharmazeutisch annehmbare Salze von Dimethylglycin. Das Salz ist besonders bevorzugt ein wasserlösliches Salz mit einer Löslichkeit in Wasser von mindestens 10 g/l bei 20°C.

In bevorzugten Ausführungsformen ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen kann das Ammoniumkation eine bis vier Alkylgruppen mit jeweils unabhängig voneinander 1 bis 4 Kohlenstoffatomen tragen. Bevorzugt sind das Natrium- und Kaliumsalz von Dimethylglycin, insbesondere das Natriumsalz von Dimethylglycin, nämlich Natrium-dimethylglycinat (Natrium-N,N-dimethylglycinat, Na-DMG).

In alternativ bevorzugten Ausführungsformen kann das Salz von Dimethylglycin das Salz einer anorganischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin, nämlich Dimethylglycin-hydrochlorid (N,N-Dimethylglycin-hydrochlorid, DMG-HCl).

Erfindungsgemäß bevorzugt ist das Dimethylglycin und/oder Salz von Dimethylglycin ausgewählt aus der Gruppe bestehend aus Dimethylglycin, Natrium-dimethylglycinat und Dimethylglycin-hydrochlorid.

Bevorzugt enthält die erfindungsgemäße Zusammensetzung 0,0001-20 Gew.-%, bevorzugt 0,01-10 Gew.-%, stärker bevorzugt 0,1-5 Gew.-% Dimethylglycin und/oder Salz von Dimethylglycin (B). In bevorzugten Ausführungsformen enthält die Zusammensetzung 0,0001-20 Gew.-%, bevorzugt 0,01-10 Gew.-%, stärker bevorzugt 0,1-5 Gew.-% Dimethylglycin, Natrium-dimethylglycinat, Dimethylglycin-hydrochlorid oder einer beliebigen Mischung davon. Bevorzugt bezieht sich diese Mengenangabe auf die Gesamtmenge aller DMG-Spezies in der Zusammensetzung.

Erfindungsgemäß ist bevorzugt, dass das Gewichtsverhältnis von Dimethylglycin und/oder Salz von Dimethylglycin (B) zu Methionin (A) in der Zusammensetzung in einem Bereich von 10:1 bis 1:1000, bevorzugt von 1:1 bis 1:100, mehr bevorzugt von 1:2 bis 1:20, insbesondere von etwa 1:3 bis etwa 1:12 liegt.

Im Rahmen der Erfindung wurde überraschend gefunden, dass die Kombination von Methionin und Dimethylglycin zu einer deutlichen Reduktion der Häufigkeit und/oder Schwere von Harnwegsinfektionen führt. Die Einnahme erwies sich insbesondere bei Personen mit Neigung zu rezidivierenden Harnwegsinfektionen als prophylaktisch wirksam.

In bevorzugten Ausführungsformen umfasst die erfindungsgemäße Zusammensetzung ferner (C) Mannose und/oder ein Mannosederivat.

Mannose ist eine Aldohexose und ein C-2-Epimer der Glucose. Die natürlich vorkommende D-Mannose spielt eine wichtige Rolle bei der Glykosylierung von Proteinen und ist Teil der Kohlenhydrat-Grundstruktur bei der *N*-Glykosylierung. Mannose kann neben der linearen Form sowohl als Fünfring (Furanose) als auch als Sechsring (Pyranose) vorliegen, wobei die Pyranoseform im Gleichgewicht deutlich überwiegt (>99%). Das Verhältnis von α-D-Mannopyranose zu β-D-Mannopyranose beträgt im Gleichgewicht in wässriger Lösung etwa 2:1. Bei der N-Glykosylierung liegt Mannose ausschließlich als Pyranosid und überwiegend in der α-Konfiguration vor. α-D-Mannopyranose ist beispielhaft in Formel (III) gezeigt:

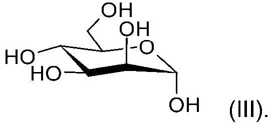

Erfindungsgemäß kann die Mannose als L-Mannose, D-Mannose oder eine Mischung davon vorliegen. D-Mannose ist erfindungsgemäß bevorzugt.

Erfindungsgemäß sind Mannosederivate bevorzugt ausgewählt aus D-Mannosiden, L-Mannosiden, Di-, Oligo- oder Polysacchariden (z.B. Pektinen) umfassend mindestens eine D- oder L-Mannoseeinheit und Mannose-ähnlichen Zuckeralkoholen. Derivate der D-Mannose sind erfindungsgemäß bevorzugt. Bevorzugt handelt es sich bei dem Mannosederivat um ein D- oder L-Mannosid, insbesondere um ein D-Mannosid.

Als Saccharide sind Di- bzw. Oligosaccharide bevorzugt, welche mindestens eine D-Mannoseeinheit, bevorzugt mindestens eine α-D-Mannopyranoseeinheit umfassen. Bevorzugte Oligosaccharide können beispielsweise bis zu 10 Zuckereinheiten umfassen und verzweigt oder linear sein. Die übrigen Zuckereinheiten sind bevorzugt Aldohexosen und/oder Derivate und sind bevorzugt ausgewählt aus Glucose, Galactose, N-Acetylglucosamin, Fucose, N-Acetylgalactosamin, Glucuronsäure und beliebigen Kombinationen davon.

Wie hierin verwendet, sind Mannose-ähnliche Zuckeralkohole bevorzugt C₆-Zuckeralkohole, welche sich insbesondere strukturell von D- oder L-Mannose oder Epimeren von D- oder L-Mannose ableiten. "Strukturell ableiten" bedeutet dabei die gleiche Konfiguration an jedem der Stereozentren (C2-C5), wie sie beispielsweise aus einer Fischer-Projektion ersichtlich ist, unabhängig von der formalen Bezeichnung der Stereozentren als *R* oder *S.* Bevorzugte Mannose-ähnliche Zuckeralkohole sind Mannitol oder Sorbitol.

Wie hierin verwendet, sind Mannoside ringförmige Mannosestrukturen, bevorzugt Mannopyranoside, deren C-1-Atom über eine glycosidische Bindung mit einem beliebigen organischen Rest R verknüpft ist. Die glycosidische Bindung kann eine *O-, S-, N-* oder *C-*glycosidische Bindung sein, bevorzugt eine O- oder S-glycosidische Bindung. Die übrigen Hydroxygruppen der Mannose liegen bevorzugt als -OH vor, gegebenenfalls können jedoch ein oder mehrere Hydroxygruppen unabhängig voneinander ersetzt sein durch H, C₁-C₁₀-Alkyl, C₁-C₁₀-Alkoxy, Halogen, NH₂, NH(C₁-C₁₀-Alkyl), N(C₁-C₁₀-Alkyl)₂, NO₂, CN, SH, SO₂(C₁-C₁₀-Alkyl) oder SO₂NH(C₁-C₁₀-Alkyl). Der organische Rest R ist bevorzugt ausgewählt aus unsubstituiertem oder substituiertem Alkyl, Cycloalkyl, Aryl, Heteroaryl, Heterocyclyl und/oder einem beliebigen Mono- oder Disaccharid. In bevorzugten Ausführungsformen umfasst das Mono- oder Disaccharid oder Derivat davon Mannose, Glucose, Galactose, N-Acetylglucosamin, Fucose, N-Acetylgalactosamin und/oder Glucuronsäure oder besteht daraus. Bevorzugt ist der organische Rest R wie unten definiert.

Bevorzugte D-Mannoside werden durch die Formeln (IIIa) und/oder (IIIb), insbesondere durch Formel (Illa) dargestellt: 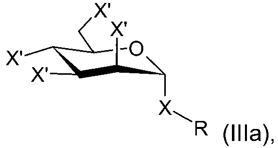 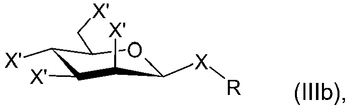 wobei
- X: ein Heteroatom ausgewählt aus O, S und N, oder Methylen, optional substituiert mit Halogen oder OH, oder eine Bindung ist,
- X': unabhängig voneinander ausgewählt ist aus OH, H, C₁-C₁₀-Alkyl, C₄-C₁₀-Alkoxy, Halogen, NH₂, NH(C₁-C₁₀-Alkyl), N(C₁-C₁₀-Alkyl)₂, NO₂, CN, SH, SO₂(C₁-C₁₀-Alkyl) und SO₂NH(C₁-C₁₀-Alkyl), und
- R: ein lineares oder verzweigtes, gesättigtes oder ungesättigtes C₁-C₂₀-Alkyl, C₅-C₁₀-Cycloalkyl, C₆-C₁₂-Aryl, Heteroaryl mit 5-12 Ringatomen, oder Heterocyclyl mit 5-12 Ringatomen, optional substituiert mit 1-3 Substituenten ausgewählt aus C₁-C₁₀-Alkyl, C₁-C₁₀-Alkoxy, -C(O)O-C₁-C₁₀-Alkyl, -C(O)NH-C₁-C₁₀-Alkyl, C₆-C₁₂-Aryl, Heteroaryl mit 5-12 Ringatomen, Heterocyclyl mit 5-12 Ringatomen, OH, Halogen, NH₂, NH(C₁-C₁₀-Alkyl), N(C₁-C₁₀-Alkyl)₂, NO₂, CN, SH, SO₂(C₁-C₁₀-Alkyl), SO₂NH(C₁-C₁₀-Alkyl), oder ein beliebiges Saccharid ist, insbesondere ein Monosachharid, ein Monosaccharid-Derivat oder ein Disaccharid ist, wobei das Saccharid bevorzugt Mannose, Glucose, Galactose, N-Acetylglucosamin, Fucose, N-Acetylgalactosamin und/oder Glucuronsäure umfasst oder daraus besteht.

Bevorzugt sind mindestens 2 X', bevorzugt mindestens 3 X', besonders bevorzugt alle X' in Formel (IIIa) und (IIIb) Hydroxygruppen (OH). Bevorzugte Halogene umfassen F, Cl, Br oder I.

In bevorzugten Ausführungsformen ist
- das Mannosid ein α-D-Mannosid der Formel (IIIa), und/oder
- X ausgewählt aus O oder S, und/oder
- X' ausgewählt aus OH, H, Me, OMe, Halogen und SH, wobei mindestens 3 X' im Mannosid OH sind, wobei bevorzugt alle X' im Mannosid OH sind, und/oder
- R ein gesättigtes, bevorzugt lineares C₁-C₂₀-Alkyl, wie z.B. n-Heptyl, ein C₆-C₁₂-Aryl, wie z.B. Biphenyl, oder ein Heteroaryl mit 5-12 Ringatomen, optional substituiert mit 1-3 Substituenten ausgewählt aus C₁-C₁₀-Alkyl, C₁-C₁₀-Alkoxy, -C(O)O-C₁-C₁₀-Alkyl, - C(O)NH-C₁-C₁₀-Alkyl, C₆-C₁₂-Aryl, Heteroaryl mit 5-12 Ringatomen, Heterocyclyl mit 5-12 Ringatomen, OH, Halogen, NH₂, NH(C₁-C₁₀-Alkyl), N(C₁-C₁₀-Alkyl)₂, NO₂, CN, SH, SO₂(C₁-C₁₀-Alkyl), SO₂NH(C₁-C₁₀-Alkyl).

In bevorzugten Ausführungsformen ist das Mannosederivat ein α-D-Mannopyranosid, welches über eine O- oder S-glycosidische Bindung mit einem organischen Rest R verknüpft ist, wobei R ausgewählt ist aus n-(C₁-C₂₀-)Alkyl, C₅-C₁₀-Cycloalkyl, (C₆-C₁₂-)Aryl, oder Heteroaryl mit 5-12 Ringatomen, bevorzugt n-(C₁-C₂₀-)Alkyl, (C₆-C₁₂-)Aryl, oder Heteroaryl mit 5-12 Ringatomen, jeweils optional substituiert mit 1-3 Substituenten ausgewählt aus C₁-C₁₀-Alkyl, C₄-C₁₀-Alkoxy, -C(O)O-C₁-C₁₀-Alkyl, -C(O)NH-C₁-C₁₀-Alkyl, C₆-C₁₂-Aryl, Heteroaryl mit 5-12 Ringatomen, Heterocyclyl mit 5-12 Ringatomen, OH, Halogen, NH₂, NH(C₁-C₁₀-Alkyl), N(C₁-C₁₀-Alkyl)₂, NO₂, CN, SH, SO₂(C₁-C₁₀-Alkyl), SO₂NH(C₁-C₁₀-Alkyl).

Besonders bevorzugte D-Mannoside umfassen n-(C₁-C₁₀-)Alkyl-α-D-mannopyranoside, (C₆-C₁₂-)Aryl-α-D-mannopyranoside, (C₆-C₁₂-)Aryl-α-D-thiomannopyranoside und/oder Heteroaryl-α-D-thiomannopyranoside, wobei das Heteroaryl 5-12 Ringatome aufweist.

In bevorzugten Ausführungsformen ist die Mannose und/oder das Mannosederivat (C) ausgewählt aus D-Mannose, L-Mannose, D-Mannosiden, L-Mannosiden oder einer Mischung davon, wobei die D-Mannoside und L-Mannoside bevorzugt wie hierin beschrieben sind. Besonders bevorzugt ist die Mannose und/oder das Mannosederivat (C) ausgewählt aus D-Mannose und/oder D-Mannosiden.

Mannosylierte Glykoproteine finden sich vielfach auf der Oberfläche von Epithelien, inklusive luminaler Epithelien der menschlichen Harnwege. Viele uropathogene Bakterien können mithilfe Lektin-haltiger Oberflächenstrukturen an solche Mannosereste binden und sich so an die Epitheloberfläche anheften. Beispielsweise tragen uropathogene *E*. *coli* an den Enden von fadenförmigen Pili das α-D-Mannosid-spezifische Lektin FimH. Ohne an eine Theorie gebunden zu sein, wird vermutet, dass Mannose und/oder Mannosederivate (C) an bakterielle Lektine wie FimH binden und so die Adhäsion an und Besiedelung der inneren Epithelien der Harnwege durch Bakterien unterdrücken können.

Es ist erfindungsgemäß bevorzugt, dass es sich bei der Mannose und/oder dem Mannosederivat (C) um einen FimH-Liganden, bevorzugt um einen FimH-Antagonisten handelt. FimH-Antagonisten können beispielsweise *in vitro* über bekannte Bindungsassays wie z.B. ELISA-basierte Assays, Fluoreszenzpolarisations (FP)-Bindungsassays, Oberflächenplasmonenresonanz (SPR) und/oder Differential Scanning Fluorimetry (DSF)-Assays und/oder über funktionale Inhibitionsassays, wie z.B. den Hemagglutination Inhibition (HAI) Assay und zellbasierteAdhäsionsassays, einschließlich durchflusszytometischer (FACS) Bindungsassays, bestimmt werden. Bevorzugte FimH-Antagonisten umfassen D-Mannoside wie oben beschrieben.

Bevorzugt enthält die erfindungsgemäße Zusammensetzung 1-80 Gew.-%, mehr bevorzugt 5-70 Gew.-% und noch mehr bevorzugt 10-60 Gew.-% Mannose und/oder Mannosederivat (C). Die Mannose und/oder das Mannosederivat (C) sind bevorzugt wie oben beschrieben.

Es ist ferner bevorzugt, dass das Gewichtsverhältnis von Methionin (A) zu Mannose und/oder Mannosederivat (C) in der Zusammensetzung in einem Bereich von 10:1 bis 1:1000, bevorzugt von 1:1 bis 1:100, mehr bevorzugt von 1:2 bis 1:20, insbesondere von etwa 1:3 bis 1:5 liegt.

Es ist ferner bevorzugt, dass das Gewichtsverhältnis von Dimethylglycin und/oder Salz von Dimethylglycin (B) zu Mannose und/oder Mannosederivat (C) in der Zusammensetzung in einem Bereich von 10:1 bis 1:1000, bevorzugt von 1:1 bis 1:100, mehr bevorzugt von 1:5 bis 1:75, insbesondere von etwa 1:8 bis 1:50 liegt.

Es wurde überraschend gefunden, dass die bevorzugte erfindungsgemäße Kombination aus Methionin (A), Dimethylglycin und/oder Salz von Dimethylglycin (B) und Mannose und/oder Mannosederivat (C) besonders effektiv zur Vorbeugung von Harnwegsinfektionen ist und insbesondere die Häufigkeit und/oder Schwere von Harnwegsinfektionen reduzieren kann. Ohne an eine Theorie gebunden zu sein, wird angenommen, dass durch die Kombination von Methionin (A), Dimethylglycin (B) und Mannose bzw. Mannosederivat (C) die Vermehrung und Adhäsion uropathogener Keime gehemmt und gleichzeitig die Immunantwort und Gewebestabilität des Urothels günstig moduliert werden kann.

In bevorzugten Ausführungsformen umfasst die erfindungsgemäße Zusammensetzung ferner
(D) mindestens ein Calciumsalz.

Das Calciumsalz (D) kann ein organisches oder anorganisches Calciumsalz sein.

Bevorzugte Calciumsalze (D) können ausgewählt sein aus Calcium-L-ascorbat, Calciumcarbonat, Calciumchlorid, Calciumsalzen der Zitronensäure, Calciumgluconat, Calciumglycerophosphat, Calciumlactat, Calciumsalzen der Orthophosphorsäure, Calciumhydroxid, Calciumoxid, Calcium-D-pantothenat, Calciumhypophosphit, Calciumacetat oder beliebigen Kombinationen davon.

In bevorzugten Ausführungsformen weist das Calciumsalz (D) eine Löslichkeit in Wasser von mindestens 1 g/l, bevorzugt mindestens 10 g/l bei 20°C und pH 7 auf.

Erfindungsgemäß ist Calciumlactat als Calciumsalz (D) besonders bevorzugt.

Ohne an eine Theorie gebunden zu sein, wird vermutet, dass das Calciumsalz (D) eine stärkende Wirkung auf die Schleimhäute ausübt und damit weiter zur Vorbeugung von Harnwegsinfektionen beitragen kann.

Bevorzugt enthält die erfindungsgemäße Zusammensetzung 0,0001-10 Gew.-%, bevorzugt 0,01-8 Gew.-%, stärker bevorzugt 0,1-6 Gew.-% Calcium, wobei das Calcium bevorzugt als Calciumsalz (D) vorliegt.

Es ist ferner bevorzugt, dass das Gewichtsverhältnis von Calcium zu Methionin (A) in der Zusammensetzung in einem Bereich von 1:1 bis 1:1000, bevorzugt von 1:1 bis 1:100, mehr bevorzugt von 1:2 bis 1:10, insbesondere von etwa 1:3 bis 1:5 liegt, wobei das Calcium bevorzugt als Calciumsalz (D) vorliegt.

In bevorzugten Ausführungsformen umfasst die erfindungsgemäße Zusammensetzung:
(A) Methionin,
(B) Dimethylglycin und/oder ein Salz von Dimethylglycin, und
(D) mindestens ein Calciumsalz,
wobei die Komponenten (A), (B) und (D) bevorzugt wie oben beschrieben sind.

In besonders bevorzugten Ausführungsformen umfasst die erfindungsgemäße Zusammensetzung:
(A) Methionin,
(B) Dimethylglycin und/oder ein Salz von Dimethylglycin,
(C) Mannose und/oder ein Mannosederivat, und
(D) mindestens ein Calciumsalz,
wobei die Komponenten (A)-(D) bevorzugt wie oben beschrieben sind.

Hierbei ist bevorzugt, dass das Gewichtsverhältnis von Calcium zu Mannose und/oder Mannosederivat (C) in der Zusammensetzung in einem Bereich von 1:1 bis 1:1000, bevorzugt von 1:5 bis 1:100, insbesondere von 1:10 bis 1:20 liegt, wobei das Calcium bevorzugt als Calciumsalz (D) vorliegt.

In bevorzugten Ausführungsformen umfasst die erfindungsgemäße Zusammensetzung ferner
(E) mindestens ein Proanthocyanidin (PAC).

Proanthocyanidine (PACs) sind polyphenolische Verbindungen, welche insbesondere in Pflanzen natürlich vorkommen. Chemisch handelt es sich bei PACs um oligomere Flavan-3-ole (Flavonoide), welche insbesondere aus zwei oder mehr Monomer-Einheiten ausgewählt aus Epicatechin, Catechin, Gallocatechin und/oder Epigallocatechin bestehen. Oligomere PACs mit 2-4 Monomereinheiten sind erfindungsgemäß bevorzugt. PACs werden je nach Verbindung der Monomere in PACs des Typs B (einfache 4→8 oder 4→6 Bindung zwischen Monomeren) oder A (zusätzliche 2→O→7 oder 2→O→5 Bindung) unterteilt. Erfindungsgemäß sind Proanthocyanidine des Typs A bevorzugt. Eine bevorzugte Untergruppe der Proanthocyanidine sind Procyanidine, welche aus Catechin- und Epicatechineinheiten bestehen. Ein erfindungsgemäß besonders bevorzugtes Proanthocyanidin ist Procyanidin A2, ein Epicatechin-Dimer mit A-Typ-Verbindung, welches natürlich unter anderem in Früchten der Cranberry vorkommt.

Erfindungsgemäß handelt es sich bei dem mindestens einen Proanthocyanidin (E) um ein oder mehrere natürlich vorkommende, bevorzugt pflanzliche Proanthocyanidine. Proanthocyanidine, welche in Früchten von Pflanzen der Gattung *Vaccinium,* wie etwa Cranberry, Preiselbeere und/oder Heidelbeere, vorkommen, sind erfindungsgemäß besonders bevorzugt.

Proanthocyanidine wirken stark antioxidativ. Darüber hinaus wurde gezeigt, dass Proanthocyanidine, insbesondere A-Typ-Proanthocyanidine, die Adhäsion von Bakterien wie uropathogenem *E. coli* an Uroepithelialzellen unterdrücken können. Es hat sich überraschend gezeigt, dass die Kombination von Proanthocyanidinen mit Methionin (A), Dimethylglycin und/oder einem Salz von Dimethylglycin (B) und optional Mannose und/oder einem Mannosederivat (C) eine deutlich verbesserte vorbeugende Wirkung gegenüber Harnwegsinfektionen aufweist als die jeweiligen Einzelkomponenten oder nur eine Teilkombination dieser Wirkstoffe.

In bevorzugten Ausführungsformen enthält die erfindungsgemäße Zusammensetzung 0,0001-10 Gew.-%, bevorzugt 0,01-8 Gew.-%, stärker bevorzugt 0,1-3 Gew.-% des mindestens einen Proanthocyanidins (E).

Es ist ferner bevorzugt, dass das Gewichtsverhältnis von Proanthocyanidin (E) zu Methionin (A) in der Zusammensetzung in einem Bereich von 1:1 bis 1:1000, bevorzugt von 1:5 bis 1:100, mehr bevorzugt von 1:5 bis 1:20 liegt.

Erfindungsgemäß können als Proanthocyanidin (E) chemisch reine Proanthocyanidine, beliebige Mischungen reiner Proanthocyanidine und/oder natürliche Proanthocyanidin-Mischungen, insbesondere Proanthocyanidin-Mischungen, die natürlich in Pflanzen vorkommen, verwendet werden.

In bevorzugten Ausführungsformen liegt das mindestens eine Proanthocyanidin (E) in der Zusammensetzung in Form eines pflanzlichen Extrakts (E') vor.

In bevorzugten Ausführungsformen umfasst die erfindungsgemäße Zusammensetzung
(E') mindestens einen pflanzlichen Extrakt.

Der pflanzliche Extrakt (E') ist ein Extrakt aus ein oder mehreren Pflanzen und enthält bevorzugt ein oder mehrere pflanzlichen Wirkstoffe. Extrakte aus PAC-haltigen Pflanzen, welche ein oder mehrere Proanthocyanidine enthalten, sind erfindungsgemäß bevorzugt. Der Extrakt kann aus beliebigem Pflanzenmaterial hergestellt sein (z.B. Früchte, Blätter, Rinde), sofern er zur oralen Anwendung geeignet ist. Die Herstellung pflanzlicher Extrakte ist dem Fachmann bekannt. Beispielsweise können pflanzliche Extrakte durch Extraktion des Pflanzenmaterials mit einem Lösungs- bzw. Extraktionsmittel, wie etwa mit Wasser, Ethanol, Propan, Butan, Ethylacetat, Kohlendioxid, Aceton, Hexan, Methylacetat, Ethylmethylketon, Dichlormethan, Methanol, Propan-2-ol, Diethylether, Butan-1-ol, Butan-2-ol und/oder Distickstoffmonoxid sowie Gemischen davon, erhalten werden.

Erfindungsgemäß ist der pflanzliche Extrakt (E') bevorzugt ein Extrakt aus Pflanzen, welche A-Typ-Proanthocyanidine enthalten. Hierzu zählen insbesondere Pflanzen der Gattungen *Vaccinium, Arachis, Cinnamomum, Litchi* und *Persea.* In bevorzugten Ausführungsformen ist der pflanzliche Extrakt (E') ein Extrakt aus Cranberry (*Vaccinium macrocarpon*), Preiselbeere (*Vaccinium vitis-idaea*), Heidelbeere (z.B. *Vaccinium myrtillus, Vaccinium corymbosum*), Erdnussschalen (*Arachis hypogaea*), Zimtrinde (z.B. *Cinnamomum verum, Cinnamomum cassia*), Litschi-Perikarp (*Litchi chinensis*), Avocado (*Persea americana*) oder Mischungen davon. Bevorzugt ist der pflanzliche Extrakt (E') reich an A-Typ- Proanthocyanidinen wie etwa Procyanidin A2. Das Vorliegen von A-Typ-Proanthocyanidinen in Pflanzen und Pflanzenteilen kann durch Analyse ihrer Extrakte mittels bekannter Methoden, beispielsweise mittels Chromatographie-basierter Methoden und/oder Massenspektrometrie-Methoden, bestimmt werden.

Extrakte aus Pflanzen der Gattung *Vaccinium,* insbesondere aus Früchten von Pflanzen der Gattung *Vaccinium* sind erfindungsgemäß besonders bevorzugt. Die Pflanzen der Gattung *Vaccinium* sind dabei bevorzugt ausgewählt aus Cranberry (*Vaccinium macrocarpon*), Preiselbeere (*Vaccinium vitis-idaea*), Heidelbeere (z.B. *Vaccinium myrtillus, Vaccinium corymbosum*) und beliebigen Mischungen davon. Cranberry (*Vaccinium macrocarpon*) ist erfindungsgemäß besonders bevorzugt.

In bevorzugten Ausführungsformen umfasst der mindestens einen pflanzlichen Extrakt (E') Cranberry-Extrakt. Besonders bevorzugt handelt es sich bei dem mindestens einen pflanzlichen Extrakt (E') um einen Cranberry-Extrakt oder um eine Mischung von Cranberry-Extrakt mit ein oder mehreren Extrakten weiterer Pflanzen der Gattung *Vaccinium,* insbesondere Preiselbeere und/oder Heidelbeere. Extrakte aus Früchten von Cranberry und gegebenenfalls Preiselbeere und/oder Heidelbeere sind erfindungsgemäß bevorzugt.

Erfindungsgemäß weist Cranberry einen charakteristischen Gehalt an A-Typ-Proanthocyanidinen auf, insbesondere A-Typ-Procyanidin-Dimeren und -Oligomeren (z.B. Procyanidin A2-Typ und A-Typ-Trimere), welche hemmend auf die bakterielle Adhäsion wirken können.

Bevorzugt enthält der pflanzliche Extrakt (E') mindestens 10 Gew.-%, bevorzugt mindestens 20 Gew.-%, stärker bevorzugt mindestens 30 Gew.-%, insbesondere mindestens 35 Gew.-% Proanthocyanidine, bezogen auf das Gesamtgewicht des pflanzlichen Extrakts. Bevorzugt handelt es sich bei den Proanthocyanidinen um Proanthocyanidine (E) wie oben beschrieben. Es ist demnach bevorzugt, dass das Proanthocyanidin (E) in der erfindungsgemäßen Zusammensetzung in Form des mindestens einen pflanzlichen Extrakts (E') vorliegt.

Es ist ferner bevorzugt, dass die Zusammensetzung 0,0001-10 Gew.-%, mehr bevorzugt 0,01-8 Gew.-%, stärker bevorzugt 0,1-6 Gew.-% pflanzlichen Extrakt (E') enthält, bezogen auf das Gesamtgewicht der Zusammensetzung.

Bevorzugt liegt das Gewichtsverhältnis von pflanzlichem Extrakt (E') zu Methionin (A) in der Zusammensetzung in einem Bereich von 1:1 bis 1:1000, bevorzugt von 1:1 bis 1:100, mehr bevorzugt von 1:2 bis 1:10, insbesondere von etwa 1:3 bis etwa 1:8.

In bevorzugten Ausführungsformen umfasst die erfindungsgemäße Zusammensetzung die Komponenten:
(A) Methionin,
(B) Dimethylglycin und/oder ein Salz von Dimethylglycin,
(C) gegebenenfalls Mannose und/oder ein Mannosederivat,
(D) gegebenenfalls mindestens ein Calciumsalz,
(E) und mindestens ein Proanthocyanidin (PAC), bevorzugt in Form eines pflanzlichen Extrakts (E'),
wobei die Komponenten (A)-(E)/(E') bevorzugt wie oben beschrieben sind. In besonders bevorzugten Ausführungsformen umfasst die erfindungsgemäße Zusammensetzung alle Komponenten (A)-(E).

In bevorzugten Ausführungsformen umfasst die erfindungsgemäße Zusammensetzung die Komponenten:
(A) Methionin,
(B) Dimethylglycin und/oder ein Salz von Dimethylglycin,
(C) gegebenenfalls Mannose und/oder ein Mannosederivat,
(D) gegebenenfalls mindestens ein Calciumsalz,
(E') und mindestens einen pflanzlichen Extrakt, bevorzugt aus ein oder mehreren Pflanzen der Gattung *Vaccinium,* insbesondere aus Cranberry, wobei der pflanzliche Extrakt bevorzugt mindestens ein Proanthocyanidin (E) umfasst,
wobei die Komponenten (A)-(D) und (E')/(E) bevorzugt wie oben beschrieben sind. In besonders bevorzugten Ausführungsformen umfasst die erfindungsgemäße Zusammensetzung alle Komponenten (A)-(D) und (E').

In weiteren bevorzugten Ausführungsformen umfasst die erfindungsgemäße Zusammensetzung
- die Komponenten (A), (B) und (E);
- die Komponenten (A), (B), (C) und (E); oder
- die Komponenten (A), (B), (D) und (E),
wobei die Komponenten (A)-(E) bevorzugt wie oben beschrieben sind.

In weiteren bevorzugten Ausführungsformen umfasst die erfindungsgemäße Zusammensetzung
- die Komponenten (A), (B) und (E');
- die Komponenten (A), (B), (C) und (E'); oder
- die Komponenten (A), (B), (D) und (E'),
wobei die Komponenten (A)-(D) und (E') bevorzugt wie oben beschrieben sind.

In bevorzugten Ausführungsformen umfasst die erfindungsgemäße Zusammensetzung ferner mindestens ein Vitamin. Der Begriff "Vitamin" ist dem Fachmann bekannt und bezieht sich wie hierin verwendet insbesondere auf organische, meist niedermolekulare Verbindungen, welche im Stoffwechsel essentielle Funktionen haben, jedoch vom menschlichen Körper nicht bedarfsdeckend synthetisiert werden und mit der Nahrung aufgenommen werden müssen.

Bevorzugte Vitamine umfassen Vitamin C, Vitamin D, Vitamin B2, Vitamin B6, Vitamin B12, Biotin (auch bekannt als Vitamin B7 oder Vitamin H) und beliebigen Kombinationen davon. Erfindungsgemäß haben Vitamine in der Zusammensetzung eine zusätzliche immunfördernde und schleimhautstärkende Wirkung und können die prophylaktische Wirkung der Zusammensetzung noch weiter verbessern.

Bevorzugt kann die erfindungsgemäße Zusammensetzung Vitamine in einer Gesamtmenge von 0,0001-10 Gew.-%, mehr bevorzugt 0,01-8 Gew.-%, stärker bevorzugt 0,1-6 Gew.-%, bezogen auf das Gesamtgewicht der Zusammensetzung, enthalten.

Bevorzugt umfasst die Zusammensetzung ferner mindestens einen Exzipienten. Bevorzugte allgemeine Exzipienten umfassen insbesondere Füll- oder Trägerstoffe, Aroma- oder Geschmacksstoffe, Farbstoffe, Konservierungsstoffe, Säureregulatoren, Antioxidantien und beliebige Mischungen davon. Als Säureregulatoren bzw. Säuerungsmittel können beliebige pharmazeutisch annehmbare Säuren eingesetzt werden, wobei Zitronensäure und/oder Ascorbinsäure bevorzugt sind. Je nach Verabreichungsform kann die Zusammensetzung noch weitere Exzipienten umfassen, wie beispielsweise Trennmittel, Bindemittel, Sprengmittel, Überzugsmittel bzw. Filmbildner (coating agents), Fließregulierungsmittel, Schmiermittel, Viskositätserhöher, Gelbildner, Lösungsvermittler bzw. Benetzungsmittel und weitere für die jeweilige Darreichungsform übliche pharmazeutische Hilfsstoffe. Erfindungsgemäß können Exzipienten die Bereitstellung in der gewünschten Verabreichungsform ermöglichen oder erleichtern, zur physikalischen und chemischen Stabilität beitragen und die orale Applizierbarkeit sowie Akzeptanz der Zusammensetzung erleichtern.

Bevorzugte Exzipienten sind insbesondere ausgewählt aus mikrokristalliner Cellulose, Lactose, Mannitol, Sorbitol, Stärke, Calciumhydrogenphosphat, Hydroxypropylcellulose, PEG, Crosscarmellose-Natrium, Crospovidon, Magnesiumstearat, Stearinsäure, Siliciumdioxid, Talkum, Tricalciumphosphat, Sucralose, Fructose, Saccharin, Glycerol, Tocopherolen, Hypromellose, Eisenoxiden, Dextrose, Inulin, Fructo-Oligosacchariden, Milchpulver, und beliebigen Mischungen davon.

Wie hierin beschrieben, umfassen die Wirkstoffe der erfindungsgemäßen Zusammensetzung insbesondere:
- die Komponenten (A) und (B), und
- bevorzugt mindestens eine Komponente, stärker bevorzugt mindestens zwei Komponenten, besonders bevorzugt alle Komponenten aus (C), (D) und [(E) und/oder (E')], und
- bevorzugt mindestens ein Vitamin.

In bevorzugten Ausführungsformen sind sämtliche Wirkstoffe in der Zusammensetzung ausgewählt aus den Komponenten (A)-(E)/(E') und Vitaminen, wie hierin definiert. Erfindungsgemäß ist ferner bevorzugt, dass die Wirkstoffe 10-90 Gew.-%, bevorzugt 25-75 Gew.-% des Gesamtgewichts der Zusammensetzung ausmachen. Bevorzugt besteht der restliche Gewichtsanteil der Zusammensetzung aus Exzipienten, wie hierin beschrieben.

Bevorzugt liegt die erfindungsgemäße Zusammensetzung als Feststoff vor. Es ist insbesondere bevorzugt, dass sich die oben genannten bevorzugten Gewichtsprozente auf die Zusammensetzung in Form eines Feststoffes beziehen. Es ist jedoch auch möglich, dass die Zusammensetzung in flüssiger Form, insbesondere als Konzentrat oder Lösung, bevorzugt in einem wässrigen Lösungsmittel, vorliegt. Bevorzugt beziehen sich die oben genannten bevorzugten Gewichtsprozente in diesen Fällen auf die gelösten Komponenten der Zusammensetzung und optional auf die gesamte flüssige Zusammensetzung inklusive des Lösungsmittels.

In bevorzugten Ausführungsformen liegt die Zusammensetzung als orale Verabreichungsform vor. Bevorzugte orale Verabreichungsformen umfassen insbesondere Pulver, Granulate, Lyophilisate, Tabletten, Brausetabletten, Filmtabletten, Konzentrate, Lutschtabletten, Schmelztabletten, Tabletten mit modifizierter Wirkstofffreisetzung, wie etwa Retardtabletten, Sublingualtabletten, Buccaltabletten, Kautabletten, Dragees und/oder Kapseln.

Bevorzugt ist die orale Verabreichungsform in getrennten Portionen bereitgestellt. Jede Portion enthält dabei bevorzugt 0,01-20 g der Zusammensetzung, insbesondere 2-7 g der Zusammensetzung. Erfindungsgemäß erleichtert die Portionierung die Dosierung und Handhabung der Zusammensetzung und kann auch zu einer verbesserten Haltbarkeit beitragen. Die getrennten Portionen können beispielsweise zur mehrmals täglichen, täglichen oder wöchentlichen Einnahme vorgesehen sein. Beispiele für Portionen der oralen Verabreichungsform umfassen verschiedene Tablettenarten, Dragees oder Kapseln wie oben beschrieben, aber auch Portionen von Konzentrat, Pulver oder Granulat, bereitgestellt in verschlossenen Verpackungen wie beispielsweise Portionsbeuteln (Sachets).

Die erfindungsgemäße Zusammensetzung und/oder orale Verabreichungsform kann zur direkten oralen Einnahme und/oder zur oralen Einnahme einer Lösung, insbesondere einer wässrigen Lösung der Zusammensetzung bzw. Verabreichungsform ausgestaltet sein.

In bevorzugten Ausführungsformen ist die Zusammensetzung oder Verabreichung zur oralen Einnahme einer wässrigen Lösung der Zusammensetzung bzw. Verabreichungsform ausgestaltet. Bevorzugt liegt die Zusammensetzung dabei in fester Form vor, welche zum Auflösen in Wasser vorgesehen ist, insbesondere als Pulver oder lösbare Tablette, beispielsweise als Brausetablette. Das Pulver ist bevorzugt portionsweise bereitgestellt, beispielsweise in Sachets.

Bevorzugt weist die Zusammensetzung oder ein überwiegender Teil der Zusammensetzung eine Löslichkeit in Wasser von mindestens 10 g/l, bevorzugt mindestens 25 g/l bei 20°C auf. Ein "überwiegender Teil" bedeutet dabei mindestens 80 Gew.-%, bevorzugt mindestens 90 Gew.-%, insbesondere mindestens 95 Gew.-% der Zusammensetzung, jeweils bezogen auf das Gesamtgewicht der Zusammensetzung.

Es ist ferner bevorzugt, dass der pH-Wert einer wässrigen Lösung der Zusammensetzung mit einer Konzentration von 30 g/l bei 20°C in einem Bereich von 3-10 liegt, bevorzugt in einem Bereich von 3,5-7, insbesondere in einem Bereich von 3,5-6 liegt.

Ein weiterer Aspekt der Erfindung betrifft eine Lösung umfassend die erfindungsgemäße Zusammensetzung und Wasser.

Bevorzugt liegt die erfindungsgemäße Zusammensetzung in der Lösung in einer Konzentration von 1-100 g/l, bevorzugt 5-80 g/l, insbesondere 20-50 g/l vor. Es ist erfindungsgemäß bevorzugt, dass die Zusammensetzung in der Lösung vollständig gelöst oder überwiegend gelöst vorliegt, wobei "überwiegend gelöst" bedeutet, dass die Zusammensetzung zu mindestens 80 Gew.-%, bevorzugt zu mindestens 90 Gew.-%, insbesondere zu mindestens 95 Gew.-% gelöst vorliegt. Gegebenenfalls vorhandene ungelöste Anteile der Zusammensetzung können erfindungsgemäß in der Lösung aufgeschwemmt und wenigstens teilweise, bevorzugt größtenteils, mit der Lösung eingenommen werden.

Bevorzugt kann die Lösung als fertige Trinklösung (ready-to-use), Konzentrat und/oder in fester Form zum Auflösen in Wasser, beispielsweise als Pulver oder Brausetablette, bereitgestellt werden. Feste Formen zum Auflösen in Wasser sind erfindungsgemäß besonders bevorzugt, insbesondere da sie eine hohe physikalische, chemische und mikrobiologische Stabilität aufweisen und zugleich gewichts- und platzsparend lager- und transportfähig sind.

Die Einnahme der Zusammensetzung in Form einer Lösung ist erfindungsgemäß besonders vorteilhaft, da die Wirkstoffkombination bereits gelöst vorliegt und somit rasch verfügbar ist, die Einnahme auch für Individuen mit Schluckbeschwerden erleichtert wird und gleichzeitig eine definierte Flüssigkeitsmenge aufgenommen wird, die zur gewünschten Erhöhung der Trinkmenge und damit zur Durchspülung der Harnwege beiträgt.

In einem weiteren Aspekt betrifft die vorliegende Erfindung die erfindungsgemäße Zusammensetzung oder Lösung zur Verwendung bei der Vorbeugung und/oder Behandlung von Infektionen und/oder Entzündungen der Harnwege. Ebenso betrifft die Erfindung ein Verfahren zur Vorbeugung und/oder Behandlung von Infektionen und/oder Entzündungen der Harnwege in einem Individuum, das dessen bedarf, umfassend Verabreichung der erfindungsgemäßen Zusammensetzung und/oder Lösung an das Individuum.

Bei den Infektionen und/oder Entzündungen der Harnwege handelt es sich bevorzugt um bakterielle Infektionen und/oder bakteriell bedingte Entzündungen, wie etwa entzündliche Veränderungen der Harnwege, welche mit bakteriellen Infektionen einhergehen können. Erfindungsgemäß wird eine breite Wirkung gegen verschiedene uropathogene Bakterien erzielt, einschließlich der häufigsten uropathogenen Bakterien wie beispielweise uropathogene *E. coli.* Die erfindungsgemäße Verwendung ist jedoch nicht auf Infektionen mit bestimmten Bakterien beschränkt.

Bei den Harnwegen handelt es sich erfindungsgemäß insbesondere um ableitende Harnwege. Erfindungsgemäß können die Infektionen und/oder Entzündungen die oberen und/oder unteren Harnwege betreffen. Die Vorbeugung und/oder Behandlung von Infektionen und/oder Entzündungen der unteren Harnwege, insbesondere von Blasenentzündungen (Zystitis) und/oder Harnröhrenentzündungen (Urethritis), ist erfindungsgemäß besonders bevorzugt.

In bevorzugten Ausführungsformen wird die Zusammensetzung oder Lösung zur Vorbeugung und/oder Behandlung rezidivierender Harnwegsinfektionen eingesetzt.

Die Verwendung zur Vorbeugung von Entzündungen und/oder Infektionen der Harnwege ist erfindungsgemäß besonders bevorzugt.

Es hat sich überraschend gezeigt, dass die Einnahme, insbesondere die regelmäßige Einnahme der erfindungsgemäßen Zusammensetzung das Risiko von Harnwegsinfektionen und insbesondere die Wiederkehrrate rezidivierenden Harnwegsinfektionen deutlich senken kann. Bei akuten Entzündungen oder Infektionen können erfindungsgemäß die Beschwerden verringert und die Heilung beschleunigt werden.

Die Dosierung der Zusammensetzung oder Lösung hängt dabei von der konkreten Verwendung ab und kann vom Fachmann leicht bestimmt werden. Die erfindungsgemäße Zusammensetzung und Lösung sind äußerst gut verträglich, sodass die tägliche Dosis nicht besonders beschränkt ist und eine regelmäßige Einnahme über einen Zeitraum von mehreren Monaten bis Jahren möglich und unproblematisch ist. Bevorzugt ist eine tägliche Einnahme von ein oder mehreren Portionen, beispielsweise 1-5 oder 1-3 Portionen der Zusammensetzung oder Lösung, insbesondere über einen Zeitraum von mindestens einer Woche, bevorzugt mindestens einem Monat.

Erfindungsgemäß kann die Zusammensetzung oder Lösung allein oder in Kombination mit mindestens einem weiteren Wirkstoff, bevorzugt mit mindestens einem Antibiotikum, angewendet werden. "In Kombination" bedeutet dabei insbesondere, dass die Zusammensetzung/Lösung und der mindestens eine weitere Wirkstoff über einen überlappenden Zeitraum angewendet werden, bevorzugt regelmäßig angewendet werden. Die Anwendung, d.h. die Verabreichung an / Einnahme durch das zu behandelnde Individuum, innerhalb des überlappenden Zeitraums kann dabei gleichzeitig oder zeitlich versetzt mit einem bestimmten oder einem beliebigen Abstand erfolgen.

Insbesondere bevorzugt ist die Kombination mit mindestens einem Antibiotikum, welches bevorzugt zur Vorbeugung und/oder Behandlung von Infektionen und/oder Entzündungen der Harnwege eingesetzt wird. Antibiotika zur Vorbeugung und/oder Behandlung von Harnwegsinfektionen sind dem Fachmann bekannt und umfassen insbesondere Fosfomycin, Nitrofurantoin, Nitroxolin, Pivmecillinam, Trimethoprim, Sulfamethoxazol, Cephalosporine, Fluorchinolone oder beliebige Kombinationen davon, wie etwa Cotrimoxazol, eine Kombination aus Trimethoprim und Sulfamethoxazol. Erfindungsgemäß kann die Zusammensetzung und/oder Lösung in Bezug auf die Antibiotikatherapie unterstützend und/oder verstärkend wirken.

### Beispiele

### Beispiel 1: Lösliche Zusammensetzung

Es wurde ein lösliches Pulver hergestellt, dass pro Portion folgende Wirkstoffe enthält:

| **Inhaltsstoff** | **Menge** |
|---|---|
| D-Mannose | 2000 mg |
| Calciumlactat Pentahydrat | entsprechend 120 mg Calcium |
| L-Methionin | 500 mg |
| Cranberry-Extrakt | 90 mg, davon 36 mg PAC |
| Vitamin C | 95 mg |
| Vitamin D | 5 µg |
| Vitamin B2 | 1 mg |
| Vitamin B6 | 1,4 mg 15 |
| Vitamin B12 | 4 µg |
| Biotin | 25 µg |
| Na-Dimethylglycin | 50 mg |

Neben den genannten Wirkstoffen enthielt das Pulver noch 2272 mg Exzipienten, insbesondere Maltodextrin als Haupthilfsstoff (ca. 1,4 g).

Eine Portion (6 g) des Pulvers wurde in 200 ml Wasser bei Raumtemperatur (20°C) gelöst. Das Pulver löste sich fast vollständig auf, insbesondere war nach dem Trinken kein Bodensatz im Glas zu sehen. Die entstandene Lösung wies einen pH-Wert von 4,5 auf.

### Beispiel 2: Anwendungsstudie

In einer 6-monatigen Anwendungsstudie nahmen 30 Frauen mit nachgewiesenen rezidivierenden Harnwegsinfektionen täglich insgesamt 1-3 Portionen des Pulvers aus Beispiel 1, jeweils gelöst in 200 ml Wasser, ein. Es konnte gezeigt werden, dass die Wiederkehrrate von Harnwegsinfektionen bei den Probandinnen um bis zu 40 % reduziert werden konnte.

Folgende Items fassen die Erfindung zusammen, ohne sie jedoch zu beschränken:
1. Zusammensetzung zur oralen Anwendung, umfassend:
   (A) Methionin, und
   (B) Dimethylglycin und/oder ein Salz von Dimethylglycin.
2. Zusammensetzung nach Item 1, wobei das Methionin (A) L-Methionin, D-Methionin oder eine beliebige Mischung davon ist, wobei das Methionin (A) bevorzugt L-Methionin ist.
3. Zusammensetzung nach einem der vorhergehenden Items, welche 0,0001-25 Gew.- %, bevorzugt 0,01-20 Gew.-%, stärker bevorzugt 1-10 Gew.-% Methionin (A) enthält, bezogen auf das Gesamtgewicht der Zusammensetzung.
4. Zusammensetzung nach einem der vorhergehenden Items, dadurch gekennzeichnet, dass das Salz von Dimethylglycin (B) ausgewählt ist aus Salzen von Dimethylglycin mit Alkalimetallen, Erdalkalimetallen, Ammonium, Ammoniumverbindungen, anorganischen Säuren oder organischen Säuren.
5. Zusammensetzung nach einem der vorhergehenden Items, welche 0,0001-20 Gew.- %, bevorzugt 0,01-10 Gew.-%, stärker bevorzugt 0,1-5 Gew.-% Dimethylglycin und/oder Salz von Dimethylglycin (B) enthält, bezogen auf das Gesamtgewicht der Zusammensetzung.
6. Zusammensetzung nach einem der vorhergehenden Items, wobei das Gewichtsverhältnis von Dimethylglycin und/oder Salz von Dimethylglycin (B) zu Methionin (A) in der Zusammensetzung in einem Bereich von 10:1 bis 1:1000, bevorzugt von 1:1 bis 1:100, mehr bevorzugt von 1:2 bis 1:20, insbesondere von etwa 1:3 bis etwa 1:12 liegt.
7. Zusammensetzung nach einem der vorhergehenden Items, ferner umfassend: (C) Mannose und/oder ein Mannosederivat.
8. Zusammensetzung nach Item 7, wobei die Mannose und/oder das Mannosederivat (C) ausgewählt ist aus D-Mannose, L-Mannose, D-Mannosiden, L-Mannosiden oder einer Mischung davon, bevorzugt D-Mannose und/oder D-Mannosiden, mehr bevorzugt D-Mannose und/oder α-D-Mannopyranosiden, insbesondere D-Mannose.
9. Zusammensetzung nach Item 7 oder 8, wobei das Mannosederivat ein D-Mannosid oder L-Mannosid, bevorzugt ein D-Mannosid ist, wobei insbesondere das Mannosederivat ein α-D-Mannopyranosid ist, welches über eine O- oder S-glycosidische Bindung mit einem organischen Rest R verknüpft ist, wobei R ausgewählt ist aus n-(C₁-C₂₀-)Alkyl, C₅-C₁₀-Cycloalkyl, (C₆-C₁₂-)Aryl, oder Heteroaryl mit 5-12 Ringatomen, optional substituiert mit 1-3 Substituenten ausgewählt aus C₁-C₁₀-Alkyl, C₁-C₁₀-Alkoxy, -C(O)O-C₁-C₁₀-Alkyl, -C(O)NH-C₁-C₁₀-Alkyl, C₆-C₁₂-Aryl, Heteroaryl mit 5-12 Ringatomen, Heterocyclyl mit 5-12 Ringatomen, OH, Halogen, NH₂, NH(C₁-C₁₀-Alkyl), N(C₁-C₁₀-Alkyl)₂, NO₂, CN, SH, SO₂(C₁-C₁₀-Alkyl), SO₂NH(C₁-C₁₀-Alkyl).
10. Zusammensetzung nach einem der Items 7-9, wobei das Mannosederivat dazu fähig ist, spezifisch an FimH zu binden.
11. Zusammensetzung nach einem der Items 7-10, welche 1-80 Gew.-%, bevorzugt 5-70 Gew.-%, mehr bevorzugt 10-60 Gew.-% Mannose und/oder Mannosederivat (C) enthält, bezogen auf das Gesamtgewicht der Zusammensetzung.
12. Zusammensetzung nach einem der Items 7-11, wobei das Gewichtsverhältnis von Methionin (A) zu Mannose und/oder Mannosederivat (C) in der Zusammensetzung in einem Bereich von 10:1 bis 1:1000, bevorzugt von 1:1 bis 1:100, mehr bevorzugt von 1:2 bis 1:20, insbesondere von etwa 1:3 bis 1:5 liegt.
13. Zusammensetzung nach einem der Items 7-12, wobei das Gewichtsverhältnis von Dimethylglycin und/oder Salz von Dimethylglycin (B) zu Mannose und/oder Mannosederivat (C) in der Zusammensetzung in einem Bereich von 10:1 bis 1:1000, bevorzugt von 1:1 bis 1:100, mehr bevorzugt von 1:5 bis 1:75, insbesondere von etwa 1:8 bis 1:50 liegt.
14. Zusammensetzung nach einem der vorhergehenden Items, ferner umfassend:
   (D) mindestens ein Calciumsalz.
15. Zusammensetzung nach Item 14, wobei das Calciumsalz (D) ausgewählt ist aus Calcium-L-ascorbat, Calciumcarbonat, Calciumchlorid, Calciumsalzen der Zitronensäure, Calciumgluconat, Calciumglycerophosphat, Calciumlactat, Calciumsalzen der Orthophosphorsäure, Calciumhydroxid, Calciumoxid, Calcium-D-pantothenat, Calciumhypophosphit, Calciumacetat oder beliebigen Kombinationen davon.
16. Zusammensetzung nach Item 14 oder 15, wobei das Calciumsalz (D) eine Löslichkeit in Wasser von mindestens 1 g/l, bevorzugt mindestens 10 g/l bei 20°C und pH 7 aufweist, insbesondere wobei es sich bei Calciumsalz (D) um Calciumlactat handelt.
17. Zusammensetzung nach einem der Items 14-16, welche 0,0001-10 Gew.-%, bevorzugt 0,01-8 Gew.-%, stärker bevorzugt 0,1-6 Gew.-% Calcium enthält, bezogen auf das Gesamtgewicht der Zusammensetzung, wobei das Calcium bevorzugt als Calciumsalz (D) vorliegt.
18. Zusammensetzung nach einem der Items 14-17, wobei das Gewichtsverhältnis von Calcium zu Methionin (A) in der Zusammensetzung in einem Bereich von 1:1 bis 1:1000, bevorzugt von 1:1 bis 1:100, mehr bevorzugt von 1:2 bis 1:10, insbesondere von etwa 1:3 bis 1:5 liegt, wobei das Calcium bevorzugt als Calciumsalz (D) vorliegt.
19. Zusammensetzung nach einem der Items 14-18, umfassend:
   (A) Methionin, und
   (B) Dimethylglycin und/oder ein Salz von Dimethylglycin,
   (C) Mannose und/oder ein Mannosederivat, und
   (D) mindestens ein Calciumsalz,
   wobei die Komponenten (A)-(D) bevorzugt gemäß den vorhergehenden Items definiert sind.
20. Zusammensetzung nach Item 19, wobei das Gewichtsverhältnis von Calcium zu Mannose und/oder Mannosederivat (C) in der Zusammensetzung in einem Bereich von 1:1 bis 1:1000, bevorzugt von 1:5 bis 1:100, insbesondere von 1:10 bis 1:20 liegt, wobei das Calcium bevorzugt als Calciumsalz (D) vorliegt.
21. Zusammensetzung nach einem der vorhergehenden Items, ferner umfassend:
   (E) mindestens ein Proanthocyanidin (PAC).
22. Zusammensetzung nach Item 21, wobei es sich bei dem mindestens einen Proanthocyanidin (E) um ein oder mehrere natürlich vorkommende, bevorzugt pflanzliche Proanthocyanidine handelt, insbesondere um Proanthocyanidine, welche in Früchten von Pflanzen der Gattung *Vaccinium,* wie etwa Cranberry, Preiselbeere und/oder Heidelbeere, vorkommen.
23. Zusammensetzung nach einem der Items 21-22, welche 0,0001-10 Gew.-%, bevorzugt 0,01-8 Gew.-%, stärker bevorzugt 0,1-3 Gew.-% Proanthocyanidin (E) enthält, bezogen auf das Gesamtgewicht der Zusammensetzung.
24. Zusammensetzung nach einem der Items 21-23, wobei das Gewichtsverhältnis von Proanthocyanidin (E) zu Methionin (A) in der Zusammensetzung in einem Bereich von 1:1 bis 1:1000, bevorzugt von 1:5 bis 1:100, mehr bevorzugt von 1:5 bis 1:20 liegt.
25. Zusammensetzung nach einem der Items 21-24, wobei das mindestens eine Proanthocyanidin (E) in der Zusammensetzung in Form eines pflanzlichen Extrakts (E') vorliegt.
26. Zusammensetzung nach einem der vorhergehenden Items, wobei die Zusammensetzung
   (E') mindestens einen pflanzlichen Extrakt
   umfasst.
27. Zusammensetzung nach Item 25 oder 26, wobei der pflanzliche Extrakt (E') ein Extrakt aus Pflanzen der Gattung *Vaccinium,* bevorzugt aus Früchten von Pflanzen der Gattung *Vaccinium* ist, wobei die Pflanzen der Gattung *Vaccinium* bevorzugt ausgewählt sind aus Cranberry, Preiselbeere, Heidelbeere und beliebigen Mischungen davon.
28. Zusammensetzung nach einem der Items 25-27, dadurch gekennzeichnet, dass der pflanzliche Extrakt (E') mindestens 10 Gew.-%, bevorzugt mindestens 20 Gew.-%, stärker bevorzugt mindestens 30 Gew.-%, insbesondere mindestens 35 Gew.-% Proanthocyanidine enthält, bezogen auf das Gesamtgewicht des pflanzlichen Extrakts.
29. Zusammensetzung nach einem der Items 25-28, welche 0,0001-10 Gew.-%, bevorzugt 0,01-8 Gew.-%, stärker bevorzugt 0,1-6 Gew.-% pflanzlichen Extrakt (E') enthält, bezogen auf das Gesamtgewicht der Zusammensetzung.
30. Zusammensetzung nach einem der Items 25-29, wobei das Gewichtsverhältnis von pflanzlichem Extrakt (E') zu Methionin (A) in der Zusammensetzung in einem Bereich von 1:1 bis 1:1000, bevorzugt von 1:1 bis 1:100, mehr bevorzugt von 1:2 bis 1:10, insbesondere von etwa 1:3 bis etwa 1:8 liegt.
31. Zusammensetzung nach einem der vorhergehenden Items, ferner umfassend mindestens ein Vitamin.
32. Zusammensetzung nach Item 31, wobei das mindestens ein Vitamin ausgewählt ist aus Vitamin C, Vitamin D, Vitamin B2, Vitamin B6, Vitamin B12, Biotin und beliebigen Kombinationen davon.
33. Zusammensetzung nach einem der vorhergehenden Items, ferner umfassend mindestens einen Exzipienten.
34. Zusammensetzung nach Item 33, wobei der Exzipient ein oder mehrere Stoffe umfasst, welche ausgewählt sind aus Füllstoffen, Aroma- bzw. Geschmacksstoffen, Farbstoffen, Konservierungsstoffen, Säureregulatoren, Antioxidantien, Trennmitteln, Bindemitteln, Sprengmitteln, Überzugsmitteln, Filmbildnern, Fließregulierungsmitteln, Schmiermitteln, Viskositätserhöhern, Gelbildnern, Lösungsvermittlern und/oder Benetzungsmitteln.
35. Zusammensetzung nach Item 33 oder 34, umfassend mindestens einen Exzipienten ausgewählt aus mikrokristalliner Cellulose, Lactose, Mannitol, Sorbitol, Stärke, Maltodextrin, Calciumhydrogenphosphat, Hydroxypropylcellulose, PEG, Crosscarmellose-Natrium, Crospovidon, Magnesiumstearat, Stearinsäure, Siliciumdioxid, Talkum, Tricalciumphosphat, Sucralose, Fructose, Saccharin, Glycerol, Tocopherolen, Hypromellose, Eisenoxiden, Dextrose, Inulin, Fructo-Oligosacchariden, Milchpulver, und beliebigen Mischungen davon.
36. Zusammensetzung nach einem der vorhergehenden Items, welche als Feststoff vorliegt.
37. Zusammensetzung nach einem der vorhergehenden Items, welche als orale Verabreichungsform, bevorzugt als Pulver, Tablette, Brausetablette, Filmtablette, Konzentrat, Lutschtablette, Kautablette, Dragee und/oder Kapsel vorliegt.
38. Zusammensetzung nach Item 37, wobei die orale Verabreichungsform in getrennten Portionen bereitgestellt ist, wobei jede Portion bevorzugt 0,01-20 g, mehr bevorzugt 2-7 g der Zusammensetzung enthält.
39. Zusammensetzung nach einem der vorhergehenden Items, wobei mindestens 80 Gew.-%, bevorzugt mindestens 90 Gew.-%, stärker bevorzugt mindestens 95 Gew.-% der Zusammensetzung eine Löslichkeit in Wasser von mindestens 10 g/l, bevorzugt mindestens 25 g/l bei 20°C aufweist.
40. Zusammensetzung nach einem der vorhergehenden Items, wobei der pH-Wert einer wässrigen Lösung der Zusammensetzung mit einer Konzentration von 30 g/l bei 20°C in einem Bereich von 3-10 liegt, bevorzugt in einem Bereich von 3,5-7, insbesondere in einem Bereich von 3,5-6 liegt.
41. Zusammensetzung nach einem der vorhergehenden Items, wobei die Zusammensetzung zur direkten oralen Einnahme und/oder zur oralen Einnahme einer wässrigen Lösung der Zusammensetzung ausgestaltet ist.
42. Lösung umfassend die Zusammensetzung nach einem der vorhergehenden Items und Wasser.
43. Lösung nach Item 42, wobei die Zusammensetzung in einer Konzentration von 1-100 g/l, bevorzugt 5-80 g/l, insbesondere 20-50 g/l vorliegt.
44. Zusammensetzung nach einem der Items 1-41 oder Lösung nach einem der Items 42-43 zur Verwendung bei der Vorbeugung und/oder Behandlung von Infektionen und/oder Entzündungen der Harnwege.
45. Zusammensetzung oder Lösung zur Verwendung nach Item 44, wobei es sich bei den Harnwegen um ableitende Harnwege handelt.
46. Zusammensetzung oder Lösung zur Verwendung nach Item 44 oder 45, wobei es sich bei den Infektionen und/oder Entzündungen der Harnwege um Infektionen und/oder Entzündungen der unteren Harnwege, insbesondere um Blasenentzündungen (Zystitis) und/oder Harnröhrenentzündungen (Urethritis) handelt.
47. Zusammensetzung oder Lösung zur Verwendung nach einem der Items 44-46, wobei es sich bei den Infektionen und/oder Entzündungen der Harnwege um bakterielle Infektionen und/oder bakteriell bedingte Entzündungen handelt.
48. Zusammensetzung oder Lösung zur Verwendung nach einem der Items 44-47, wobei es sich bei den Infektionen und/oder Entzündungen der Harnwege um rezidivierende Harnwegsinfektionen handelt.
49. Zusammensetzung oder Lösung zur Verwendung nach einem der Items 44-48, wobei die Verwendung in Kombination mit mindestens einem Antibiotikum erfolgt.
50. Zusammensetzung oder Lösung zur Verwendung nach Item 49, wobei das mindestens eine Antibiotikum ausgewählt ist aus Fosfomycin, Nitrofurantoin, Nitroxolin, Pivmecillinam, Trimethoprim, Sulfamethoxazol, Cephalosporinen, Fluorchinolonen oder beliebige Kombinationen davon, wie etwa Cotrimoxazol.
51. Verfahren zur Vorbeugung und/oder Behandlung von Infektionen und/oder Entzündungen der Harnwege in einem Individuum, das dessen bedarf, umfassend Verabreichung der erfindungsgemäßen Zusammensetzung und/oder Lösung an das Individuum.
52. Verfahren nach Item 51, wobei die Infektionen und/oder Entzündungen der Harnwege gemäß einem der Items 46-48 definiert sind.
53. Verfahren nach Item 51 oder 52, ferner umfassend Verabreichung mindestens eines Antibiotikums an das Individuum, wobei das mindestens eine Antibiotikum bevorzugt ausgewählt ist aus Fosfomycin, Nitrofurantoin, Nitroxolin, Pivmecillinam, Trimethoprim, Sulfamethoxazol, Cephalosporinen, Fluorchinolonen oder beliebige Kombinationen davon, wie etwa Cotrimoxazol.

## Patentansprüche

1. Zusammensetzung zur oralen Anwendung, umfassend:
(A) Methionin, und
(B) Dimethylglycin und/oder ein Salz von Dimethylglycin.

2. Zusammensetzung nach Anspruch 1, wobei das Methionin (A) L-Methionin, D-Methionin oder eine beliebige Mischung davon ist, wobei das Methionin (A) bevorzugt L-Methionin ist, und/oder
wobei die Zusammensetzung 0,0001-25 Gew.-%, bevorzugt 0,01-20 Gew.-%, stärker bevorzugt 1-10 Gew.-% Methionin (A) enthält, bezogen auf das Gesamtgewicht der Zusammensetzung.

3. Zusammensetzung nach einem der vorhergehenden Ansprüche, welche 0,0001-20 Gew.-%, bevorzugt 0,01-10 Gew.-%, stärker bevorzugt 0,1-5 Gew.-% Dimethylglycin und/oder Salz von Dimethylglycin (B) enthält, bezogen auf das Gesamtgewicht der Zusammensetzung, und/oder
wobei das Gewichtsverhältnis von Dimethylglycin und/oder Salz von Dimethylglycin (B) zu Methionin (A) in der Zusammensetzung in einem Bereich von 10:1 bis 1:1000, bevorzugt von 1:1 bis 1:100, mehr bevorzugt von 1:2 bis 1:20, insbesondere von etwa 1:3 bis etwa 1:12 liegt.

4. Zusammensetzung nach einem der vorhergehenden Ansprüche, ferner umfassend:
(C) Mannose und/oder ein Mannosederivat,
wobei die Mannose und/oder das Mannosederivat (C) bevorzugt ausgewählt ist aus D-Mannose, L-Mannose, D-Mannosiden, L-Mannosiden oder einer Mischung davon, bevorzugt D-Mannose und/oder D-Mannosiden, mehr bevorzugt D-Mannose und/oder α-D-Mannopyranosiden, insbesondere D-Mannose, und/oder
wobei die Zusammensetzung bevorzugt 1-80 Gew.-%, bevorzugt 5-70 Gew.-%, mehr bevorzugt 10-60 Gew.-% Mannose und/oder Mannosederivat (C) enthält, bezogen auf das Gesamtgewicht der Zusammensetzung.

5. Zusammensetzung nach einem der Ansprüche 1-4, wobei das Gewichtsverhältnis von Methionin (A) zu Mannose und/oder Mannosederivat (C) in der Zusammensetzung in einem Bereich von 10:1 bis 1:1000, bevorzugt von 1:1 bis 1:100, mehr bevorzugt von 1:2 bis 1:20, insbesondere von etwa 1:3 bis 1:5 liegt, und/oder wobei das Gewichtsverhältnis von Dimethylglycin und/oder Salz von Dimethylglycin (B) zu Mannose und/oder Mannosederivat (C) in der Zusammensetzung in einem Bereich von 10:1 bis 1:1000, bevorzugt von 1:1 bis 1:100, mehr bevorzugt von 1:5 bis 1:75, insbesondere von etwa 1:8 bis 1:50 liegt.

6. Zusammensetzung nach einem der vorhergehenden Ansprüche, ferner umfassend:
(D) mindestens ein Calciumsalz,
wobei das Calciumsalz (D) bevorzugt ausgewählt ist aus Calcium-L-ascorbat, Calciumcarbonat, Calciumchlorid, Calciumsalzen der Zitronensäure, Calciumgluconat, Calciumglycerophosphat, Calciumlactat, Calciumsalzen der Orthophosphorsäure, Calciumhydroxid, Calciumoxid, Calcium-D-pantothenat, Calciumhypophosphit, Calciumacetat oder beliebigen Kombinationen davon, und/oder wobei das Calciumsalz (D) bevorzugt eine Löslichkeit in Wasser von mindestens 1 g/l, bevorzugt mindestens 10 g/l bei 20°C und pH 7 aufweist.

7. Zusammensetzung nach einem der Ansprüche 1-6, welche 0,0001-10 Gew.-%, bevorzugt 0,01-8 Gew.-%, stärker bevorzugt 0,1-6 Gew.-% Calcium enthält, bezogen auf das Gesamtgewicht der Zusammensetzung, wobei das Calcium bevorzugt als Calciumsalz (D) vorliegt, und/oder
wobei das Gewichtsverhältnis von Calcium zu Methionin (A) in der Zusammensetzung in einem Bereich von 1:1 bis 1:1000, bevorzugt von 1:1 bis 1:100, mehr bevorzugt von 1:2 bis 1:10, insbesondere von etwa 1:3 bis 1:5 liegt, wobei das Calcium bevorzugt als Calciumsalz (D) vorliegt.

8. Zusammensetzung nach einem der vorhergehenden Ansprüche, ferner umfassend:
(E) mindestens ein Proanthocyanidin (PAC).

9. Zusammensetzung nach einem der Ansprüche 1-8, welche 0,0001-10 Gew.-%, bevorzugt 0,01-8 Gew.-%, stärker bevorzugt 0,1-3 Gew.-% Proanthocyanidin (E) enthält, bezogen auf das Gesamtgewicht der Zusammensetzung, und/oder wobei das Gewichtsverhältnis von Proanthocyanidin (E) zu Methionin (A) in der Zusammensetzung in einem Bereich von 1:1 bis 1:1000, bevorzugt von 1:5 bis 1:100, mehr bevorzugt von 1:5 bis 1:20 liegt.

10. Zusammensetzung nach einem der Ansprüche 1-9, wobei das mindestens eine Proanthocyanidin (E) in der Zusammensetzung in Form eines pflanzlichen Extrakts vorliegt,
wobei der pflanzliche Extrakt bevorzugt ein Extrakt aus Pflanzen der Gattung *Vaccinium,* bevorzugt aus Früchten von Pflanzen der Gattung *Vaccinium* ist, und/oder wobei der pflanzliche Extrakt bevorzugt mindestens 10 Gew.-%, stärker bevorzugt mindestens 20 Gew.-%, stärker bevorzugt mindestens 30 Gew.-%, insbesondere mindestens 35 Gew.-% Proanthocyanidine enthält, bezogen auf das Gesamtgewicht des pflanzlichen Extrakts.

11. Zusammensetzung nach einem der vorhergehenden Ansprüche, ferner umfassend mindestens ein Vitamin und/oder mindestens einen Exzipienten,
wobei das mindestens ein Vitamin bevorzugt ausgewählt ist aus Vitamin C, Vitamin D, Vitamin B2, Vitamin B6, Vitamin B12, Biotin und beliebigen Kombinationen davon, wobei der mindestens eine Exzipient bevorzugt ein oder mehrere Stoffe umfasst, welche ausgewählt sind aus Füllstoffen, Aroma- bzw. Geschmacksstoffen, Farbstoffen, Konservierungsstoffen, Säureregulatoren, Antioxidantien, Trennmitteln, Bindemitteln, Sprengmitteln, Überzugsmitteln, Filmbildnern, Fließregulierungsmitteln, Schmiermitteln, Viskositätserhöhern, Gelbildnern, Lösungsvermittlern und/oder Benetzungsmitteln.

12. Zusammensetzung nach einem der vorhergehenden Ansprüche, welche als Feststoff vorliegt, und/oder
welche als orale Verabreichungsform, bevorzugt als Pulver, Tablette, Brausetablette, Filmtablette, Konzentrat, Lutschtablette, Kautablette, Dragee und/oder Kapsel vorliegt, und/oder
wobei die Zusammensetzung zur direkten oralen Einnahme und/oder zur oralen Einnahme einer wässrigen Lösung der Zusammensetzung ausgestaltet ist.

13. Zusammensetzung nach einem der vorhergehenden Ansprüche, wobei mindestens 80 Gew.-%, bevorzugt mindestens 90 Gew.-%, stärker bevorzugt mindestens 95 Gew.-% der Zusammensetzung eine Löslichkeit in Wasser von mindestens 10 g/l, bevorzugt mindestens 25 g/l bei 20°C aufweist, und/oder
wobei der pH-Wert einer wässrigen Lösung der Zusammensetzung mit einer Konzentration von 30 g/l bei 20°C in einem Bereich von 3-10 liegt, bevorzugt in einem Bereich von 3,5-7, insbesondere in einem Bereich von 3,5-6 liegt.

14. Lösung umfassend die Zusammensetzung nach einem der vorhergehenden Ansprüche und Wasser,
wobei die Zusammensetzung in der Lösung bevorzugt in einer Konzentration von 1-100 g/l, bevorzugt 5-80 g/l, insbesondere 20-50 g/l vorliegt.

15. Zusammensetzung nach einem der Ansprüche 1-13 oder Lösung nach Anspruch 14 zur Verwendung bei der Vorbeugung und/oder Behandlung von Infektionen und/oder Entzündungen der Harnwege,
wobei es sich bei den Infektionen und/oder Entzündungen der Harnwege bevorzugt um Infektionen und/oder Entzündungen der unteren Harnwege, und/oder um bakterielle Infektionen und/oder bakteriell bedingte Entzündungen, und/oder um rezidivierende Harnwegsinfektionen handelt.
